# NEUE EUROPÄISCHE PATENTSCHRIFT

(11) **EP 1 716 882 B2**
(45) Veröffentlichungstag und Bekanntmachung des Hinweises auf die Entscheidung über den Einspruch: **08.08.2018**
(45) Hinweis auf die Patenterteilung: 13.08.2008
(21) Anmeldenummer: 06012884.0
(22) Anmeldetag: 26.02.2002
(51) Int. Cl.: A61M 25/06, A61M 5/32

(54) **Schutzvorrichtung für eine Injektionsnadel**
Protection device for an injection needle
Dispositif de protection pour une aiguille d'injection

(30) Priorität: 26.02.2001 DE 20103363 U
(43) Veröffentlichungstag der Anmeldung: 02.11.2006
(62) Teilanmeldung aus: 02719930.6
(73) Patentinhaber: B. Braun Melsungen AG, 34212 Melsungen (DE)
(72) Erfinder: Woehr, Kevin, 34587 Felsberg (DE); Fuchs, Jürgen, 34308 Bad Emstal (DE)
(74) Vertreter: Kinkeldey, Daniela

(56) Entgegenhaltungen:
- EP-A- 0 554 841
- EP-A- 0 638 326
- EP-A2- 0 750 915
- EP-A2- 0 750 917
- WO-A-99/08742
- WO-A1-00/69501
- DE-T2- 69 106 088
- US-A- 5 049 136
- US-A- 5 322 517

## Beschreibung

Die Erfindung betrifft eine Schutzvorrichtung für eine Injektions- bzw. Infusionsnadel nach dem Oberbegriff des Anspruchs 1.

Eine Schutzvorrichtung dieser Art ist aus EP-A-0 554 841 bekannt, wobei das Griffteil seitlich offen ist und der in der Bereitstellung auf dem Nadelschaft abgestützte Arm des Schutzelementes seitlich aus dem Griffteil hervorragt. Von der proximalen Rückwand des Schutzelementes ragt ein Gabelabschnitt in distale Richtung, der in einer Ausnehmung des Griffteils so eingeklemmt ist, dass der gegenüberliegende Arm des Schutzelementes unter Federspannung in der Bereitstellung am Nadelschaft anliegt.

Weiterhin ist aus EP-A-0 638 326 eine Schutzvorrichtung bekannt, verschiedene Ausführungsformen eines Schutzelements beschrieben werden, das von einem äußeren Gehäuse umgeben ist, das durch erhöhte Kraft in distale Richtung von dem in der Schutzstellung auf der Nadel gehaltenen Schutzelement abgezogen werden kann. Bei der einen Ausführungsform weist das Schutzelement zwei sich in distale Richtung erstreckende Arme auf, wobei das Schutzelement durch radial nach innen vorstehende Spreizelemente mit dem Nadelumfang in Eingriff tritt, um das Schutzelement in der Schutzstellung zu halten. Bei einer anderen Ausführungsform ist in dem äußeren Gehäuse ein geschlossenes Schutzelement mit einem inneren Gehäuse angeordnet, das verschiedene Bauelemente des Schutzelementes aufnimmt und zusammenhält. Hierbei wird von einer Schraubenfeder ein einarmiges Element so beaufschlagt, dass es in der Schutzstellung einen Austritt der Nadelspitze aus dem geschlossenen inneren Gehäuse in distale Richtung verhindert, und durch Verkanten auf dem Nadelumfang so gehalten wird, dass die Nadel in proximale Richtung nicht aus dem Schutzelement herausgezogen werden kann.

Weiterhin ist eine Schutzvorrichtung aus US 4 929 241 bekannt, wobei ein relativ kleines Schutzelement auf der Nadel angeordnet ist, das mittels einer Feder aus der zurückgezogenen Stellung in die Schutzstellung an der Nadelspitze verschoben werden kann, wobei elastische Arme des Schutzelementes die Nadelspitze übergreifen, während eine Eingriffseinrichtung am Schutzelement dieses auf dem Nadelschaft hält. Aufgrund der geringen Größe des Schutzelementes ist es schwierig, dieses von Hand auf der Nadel zu verschieben. Zudem kann die Befestigungsfeder erst dann ausgelöst werden, wenn die Nadelspitze frei liegt, so dass eine Verletzungsgefahr nicht ausgeschlossen werden kann.

Der Erfindung liegt die Aufgabe zugrunde, eine Schutzvorrichtung der eingangs angegebenen Art so auszubilden, dass eine Betätigung von Hand erleichtert wird und eine Verletzungsgefahr ausgeschlossen werden kann.

Diese Aufgabe wird erfindungsgemäß durch die Merkmale des Anspruchs 1 gelöst. Dadurch, dass ein Griffteil zwischen Schutzelement und Nadelhalter zum Verschieben des Schutzelementes vorgesehen ist, kann beim Zurückziehen der Nadel mittels des Nadelhalters nach einer Injektion mit der anderen Hand das Griffteil bequem gehalten werden, so dass durch die Relativbewegung zwischen Nadel und Griffteil das Schutzelement in die Schutzposition an der Nadelspitze verschoben wird, ohne dass das kleine Schutzelement mit den Fingern berührt werden muss. Dadurch, dass das Schutzelement während des Zurückziehens der Nadel in die Schutzposition gebracht wird, kann auch eine Verletzungsgefahr ausgeschlossen werden.

Beispielsweise Ausführungsformen der Erfindung werden nachfolgend anhand der Zeichnungen näher erläutert. Es zeigen
Fig. 1 in einem Längsschnitt eine nicht beanspruchte Schutzvorrichtung,
Fig. 2 eine Seitenansicht der Ausführungsform nach Fig. 1,
Fig. 3 eine Ansicht der Vorrichtung nach den Fig. 1 und 2 mit in die Schutzstellung verschobenem Schutzelement,
Fig. 4 eine erfindungsgemäße Ausführungsform der Vorrichtung,
Fig. 5 eine Ausführungsform in Verbindung mit einem mit Flügeln versehenen Nadelhalter,
Fig. 6 eine Schnittansicht der Ausführungsform nach Fig. 5,
Fig. 7 eine Stirnansicht in Fig. 5,
Fig. 8 eine Draufsicht auf eine Ausführungsform nach Fig. 5 mit Nadelkappe,
Fig. 9 eine Ansicht der Nadelkappe nach Fig. 8,
Fig. 10 eine Draufsicht auf eine Ausführungsform nach Fig. 7 mit einer abgewandelten Nadelkappe,
Fig. 11 eine Seitenansicht der Nadelkappe nach Fig. 12,
Fig. 12 eine Schnittansicht durch eine andere Ausführungsform mit gekrümmter Nadel,
Fig. 13 eine andere Nadelkappe bei der Ausführungsform nach Fig. 12,
Fig. 14 eine Ausführungsform mit verformbarem Griffteil in der Ausgangsstellung, und
Fig. 15 das Griffteil von Fig. 14 in der gestreckten Stellung.

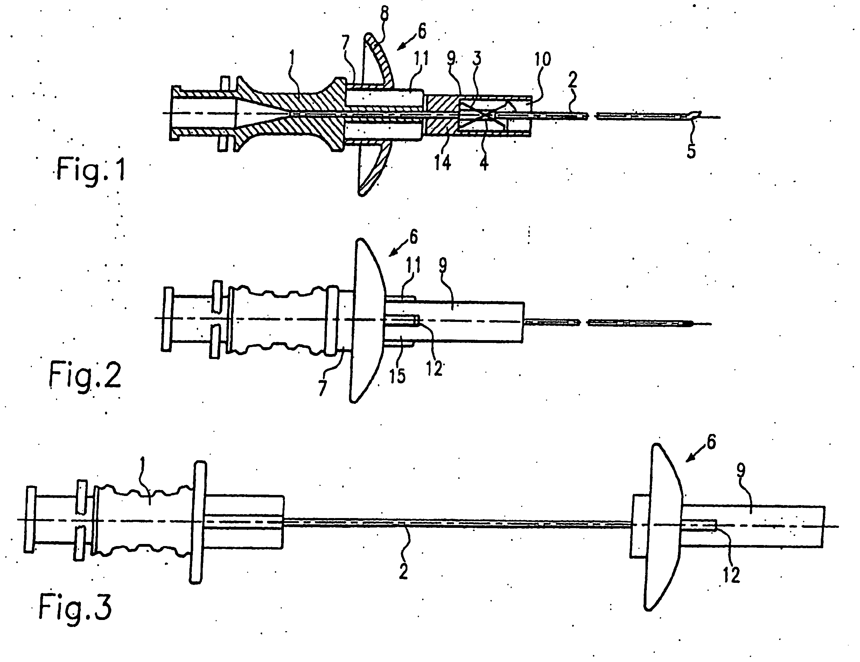

Fig. 1 zeigt einen Nadelhalter 1, in dem eine Nadel 2 befestigt ist. Auf dem Schaft der Nadel 2 ist ein Schutzelement 3 in Form eines Federclips mit sich kreuzenden Armen angeordnet. Mit 4 ist eine Hülse bezeichnet, die mit dem Schutzelement 3 längs des Nadelschaftes verschiebbar ist. Bei dem dargestellten Ausführungsbeispiel ist die Spitze 5 der Nadel entsprechend einer Epidural-Nadel oder einer Huber-Nadel gekrümmt ausgebildet, so dass die Hülse 4, die einen kleineren Durchmesser als die Krümmung an der Nadelspitze aufweist, und mit ihr das Schutzelement 3 nicht über die Nadelspitze hinaus verschoben werden kann.

Zwischen Nadelhalter 1 und Schutzelement 3 ist ein Griffteil 6 angeordnet, das am proximalen Ende einen hohlzylindrischen Abschnitt 7 aufweist, an dem ein radial abstehender Schild 8 ausgebildet ist. Auf der Vorderseite des Schildes 8 ist ein zylindrischer Abschnitt 9 ausgebildet, dessen distales Ende hohl ausgebildet ist. In dem Hohlraum 10 ist in der Bereitstellung nach Fig. 1 das Schutzelement 3 angeordnet, das durch Verschieben des Griffteiles 6 nach vorne zur Nadelspitze 5 verschoben werden kann, während mit der anderen Hand der Nadelhalter 1 gehalten wird. Hierbei übergreifen die abgewinkelten Enden der sich kreuzenden Arme des Schutzelementes 3 die Nadelspitze 5, so dass eine Verletzung der Bedienungsperson durch die Nadelspitze verhindert wird.

Der Nadelhalter 1 weist am distalen Ende radial abstehende Rippen 11 auf, auf denen der hohlzylindrische Abschnitt 7 des Griffteiles 6 geführt ist. Zwischen dem zylindrischen Abschnitt 9 mit kleinerem Außendurchmesser und dem hohlzylindrischen Abschnitt 7 mit größerem Außendurchmessers sind in dem Griffteil 6 Schlitze 12 ausgebildet, durch welche die vorderen Enden der Rippen 11 des Nadelhalters 1 radial vorstehen, wie Fig. 2 zeigt.

Der mit dem Hohlraum 10 versehene zylindrische Abschnitt 9 des Griffteils 6 weist einen vollzylindrischen Abschnitt 14 zwischen den Schlitzen 12 und dem Hohlraum 10 auf, in dessen zentrischer Bohrung die Nadel 2 geführt ist. Zwischen den Schlitzen 12 des Griffteils 6 ist der zylindrische Abschnitt 9 über Stege 15 mit dem Schild 8 bzw. dem hohlrylindrischen Abschnitt 7 einstükkig verbunden.

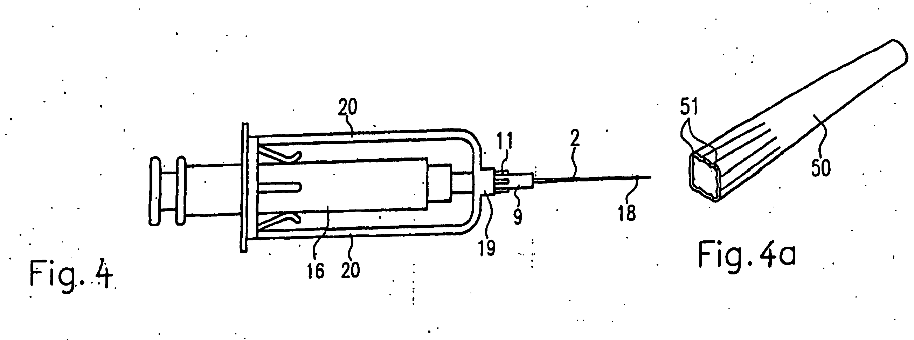

Diese über den Außenumfang des zylindrischen Abschnittes 9 des Griffteiles 6 vorstehenden Rippen 11 dienen zum Aufstecken einer Nadelkappe 13, die in Fig. 4 gezeigt ist. Diese Nadelkappe 13 wird für die Lagerung und die Handhabung der Vorrichtung verwendet. Sie kann unmittelbar vor Gebrauch der Injektionsnadel vom Nadelhalter 1 abgezogen werden, um die Nadel freizulegen, ohne dass dadurch das Griffteil 6 und das Schutzelement 3 bewegt wird, weil die Nadelkappe 13 durch die Rippen 11 in einem radialen Abstand von dem Abschnitt 9 des Griffteils 6 gehalten wird.

Aufgrund des kleineren Durchmessers am Abschnitt 14 gegenüber dem größeren Durchmesser an den Rippen 11 kann die Nadelkappe 13, die aus einem Schlauchstück mit durchgehend gleichem Durchmesser besteht, nicht auf dem Abschnitt 14 fehlpositioniert, sondern nur auf die Rippen 11 aufgesteckt werden. Hierdurch wird verhindert, dass ein Eingriff der Nadelkappe 13 mit einem Abschnitt des Griffteils 6 unbeabsichtigt hergestellt wird. Die Nadelkappe 13 kann durch Extrudieren eines Schlauches kostengünstig gefertigt werden, wobei ein Stück eines solchen Schlauches die Nadelkappe 13 bildet.

Nach Abnehmen der Nadelkappe 13 kann in der Bereitstellung nach den Fig. 1 und 2 eine Injektion ausgeführt werden, worauf beim Zurückziehen der Nadel mit einer Hand am Nadelhalter 1 mit der anderen Hand das Griffteil 6 am Abschnitt 7 gehalten wird, so dass durch die Relativbewegung zwischen Griffteil 6 und Nadel 2 das Schutzelement 3 in die Schutzstellung an der Nadelspitze verschoben wird. Diese ausgefahrene Stellung des Griffteiles 6 ist in Fig. 3 wiedergegeben.

Das Schutzelement 3 ist in dem Hohlraum 10 des Griffteils 6 lose angeordnet, so dass das Griffteil 6 aus der Stellung in Fig. 3 ohne weiteres zurückgezogen werden kann, während das Schutzelement in der Schutzstellung an der Nadelspitze verbleibt. Der Hohlraum 10 im zylindrischen Abschnitt 9 schützt das Schutzelement 3 nach Abziehen der Nadelkappe 13.

Bei der Schutzvorrichtung nach Fig. 1 und 2 dient der hohlzylindrische Abschnitt 7 am Griffteil 6 dazu, die Finger der das Griffteil haltenden Hand vor einer Berührung mit dem Nadelschaft zu schützen, wenn die Nadel zurückgezogen wird.

Bei einer anderen Ausgestaltung des Nadelhalters 1 kann dieser hohlzylindrische Abschnitt 7 hinter dem Schild 8 vergrößert ausgebildet sein.

Das Griffteil 6 wird - wie auch der Nadelhalter 1 - zweckmäßigerweise aus Kunststoff gefertigt.

Fig. 4 zeigt eine Ausführungsform eines Griffteils 6 in Verbindung mit einer Spritze 16, an der über einen Nadelhalter 17, der als Kanülenansatz ausgebildet ist, eine Injektionsnadel 2 fest angebracht ist. Bei dieser Ausführungsform ist vor der Nadelspitze ein Wulst 18 auf dem Außenumfang der Nadel ausgebildet, an dem die Rückwand des Schutzelementes 3 in der Schutzstellung zum Anliegen kommt. Anstelle eines Wulstes 18 können durch eine Nadelquetschung auch diametral gegenüberliegende noppenartige Vorsprünge ausgebildet werden.

Das Griffteil 6 weist einen zylindrischen Abschnitt 19 auf, der in der Ausgangsstellung nach Fig. 4 auf dem Nadelhalter 17 geführt ist. Von diesem zylindrischen Abschnitt 19 erstrecken sich bei dem dargestellten Ausführungsbeispiel an diametral gegenüberliegenden Stellen zwei Bügel 20 in einem Abstand vom Spritzenumfang in proximale Richtung. Die Enden dieser Bügel 20 sind an einem Ringkörper 21 angeformt, von dem aus sich elastische Finger 22 radial nach innen erstrecken. Die freien Enden dieser elastischen Finger 22 liegen auf dem Außenumfang der Spritze 16 an.

Aufgrund der elastischen Finger 22 zwischen Griffteil 6 und Außenumfang der Spritze 16 kann das Griffteil 6 für unterschiedlich große Spritzendurchmesser verwendet werden, z. B. können Spritzen mit einem Volumen von 1 ml bis 10 ml in das gleiche Griffteil eingesetzt werden. Hierdurch ist eine große Auswahl von Spritzen möglich, die mit der gleichen Nadel verwendet werden können.

Auch bei der Ausführungsform nach Fig. 4 sind am vorderen Ende des als Kanülenansatz ausgebildeten Nadelhalters radial abstehende Rippen 11 ausgebildet werden, die als Sitz für eine Nadelkappe dienen. Durch den Innenumfang des zylindrischen Abschnitts 19 wird das Schutzelement 3, dessen Rückwand über den Querschnitt der Rippen 11 vorsteht, nach vorne in die Schutzstellung verschoben.

Fig. 4 zeigt eine Ausführungsform des Griffteils 6, bei der an dem zylindrischen Abschnitt 19 ein weiterer zylindrischer Abschnitt 9 angeformt ist, in dessen Hohlraum 10 das Schutzelement 3 aufgenommen wird. Wie bei der Ausführungsform nach den Fig. 1 und 2 sind zwischen zylindrischem Abschnitt 19 und Abschnitt 9 in Achsrichtung verlaufende Schlitze ausgebildet, durch die die am Nadelhalter bzw. Kanülenansatz 17 ausgebildeten Rippen 11 zur Aufnahme der Nadelkappe 13 vorstehen.

Fig. 4a zeigt in perspektivischer Ansicht eine durch Spritzgießen ausgebildete Nadelkappe 50, deren distales Ende geschlossen sein kann, während das proximale Ende auf dem Innenumfang entsprechend der Anzahl der Rippen 11 Rillen oder Nuten 51 aufweist, die beim Aufstecken der Nadelkappe auf den Nadelhalter 17 mit den Rippen 11 in Eingriff treten, so dass durch Verdrehen der aufgesteckten Nadelkappe 50 auch der Nadelhalter 17 verdreht werden kann. Zwischen Nadelhalter 17 und Spritze 16 wird üblicherweise ein Gewindeeingriff vorgesehen, so dass durch Verdrehen der Nadelkappe 50 der Nadelhalter 17 auf die Spritze 16 aufgeschraubt werden kann.

Es ist üblich, mittels einer Nadel mit relativ großem Durchmesser Flüssigkeit in die Spritze anzusaugen und dann die Nadel gegen eine Nadel mit einem relativ kleinen Durchmesser auszuwechseln, um eine Infusion am Patienten auszuführen. Bei der Ausführungsform nach Fig. 4 kann die Nadel ohne weiteres ausgewechselt werden. Die beschriebene Ausgestaltung ermöglicht eine Betätigung mit einer Hand, wenn der Spritzeninhalt injiziert ist, wobei die Spritze 16 mit zwei Fingern gehalten und die Nadel aus der Haut des Patienten gezogen wird, während gleichzeitig sich ein Finger der Hand an dem am proximalen Ende liegenden Ringkörper 21 abstützt.

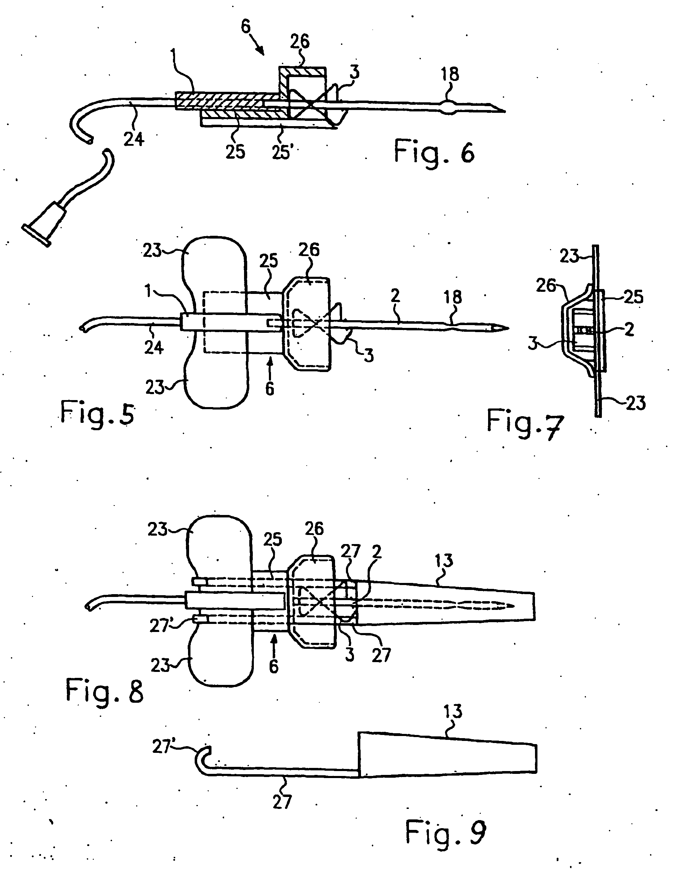

Fig. 5 zeigt in einer Draufsicht einen mit seitlich abstehenden Flügeln 23 versehenen Nadelhalter 1, an den ein Verbindungsschlauch 24 angeschlossen ist. Zwischen dem auf dem Nadelschaft angeordneten Schutzelement 3 und Nadelhalter 1 ist ein Griffteil 6 mit einem haubenförmigen Abschnitt 26 angeordnet, das wegen des flachen Injektionswinkels (Fig. 6) zweckmäßigerweise ein Flächenstück 25 zur Auflage auf der Haut des Patienten aufweist, das auf der Auflageseite beispielsweise mit einer Klebeschicht zur besseren Halterung auf der Haut versehen sein kann. Vorzugsweise wird ein Schaummaterial 25' auf der Auflageseite vorgesehen. Der von dem vorderen Ende des Flächenstücks 25 vorstehende Haubenabschnitt 26 des Griffteils 6 überdeckt wenigstens teilweise das Schutzelement 3. Das Flächenstück 25 bzw. das weiche Auflageteil 25' dient auch als Abstandhalter für das Schutzelement 3 von der Haut des Patienten. Bei dem Ausführungsbeispiel nach Fig. 6 erstreckt sich der weiche Auflageteil 25' über das Flächenstück 25 unter den Haubenabschnitt 26, so dass das Schutzelement 3 nicht auf der Haut des Patienten aufliegt.

Der mit Flügeln 23 versehene Nadelhalter 1 wird für Veneninfusionen verwendet, wobei üblicherweise eine dünne Nadel verwendet wird. Die Flügel 23 sind relativ groß und flexibel. Sie werden zusammengedrückt, wenn die Nadel in einem sehr flachen Winkel in die Haut eingeführt wird. Ein auf der Klebeschicht an der Auflagefläche aufgebrachtes, nicht dargestelltes Schutzpapier sollte nicht abgezogen werden, bis die Nadel in der Vene eingeführt ist. Nachdem die Nadel in die Vene eingeführt ist, werden die Flügel 23 auf der Haut des Patienten flach gelegt und mit einem Klebeband gehalten. Auch das Griffteil 6 kann mittels eines Klebebandes festgelegt werden, wobei der haubenförmige Abschnitt 26 einen Kontakt zwischen Schutzelement 3 und Klebeband verhindert. Beim Zurückziehen der Nadel nach Entfernen des Klebebandes vom Nadelhalter bleibt zunächst das Griffteil 6 mit dem Schutzelement 3 in seiner Stellung. Nachdem die zurückgezogene Nadelspitze durch das Schutzelement 3 sicher abgedeckt ist, wobei die Vorsprünge 18 an der Nadel das Schutzelement 3 an der Nadelspitze fixieren, kann auch das Griffteil 6 von der Haut des Patienten abgenommen werden.

Fig. 5 und 6 zeigen die Vorrichtung in der Bereitstellung zum Einführen der Nadel. Wenn die mit einer Klebeschicht versehene Auflage 25 am Griffteil verwendet wird, handelt es sich um ein passives System.

Fig. 7 zeigt eine Ansicht des Griffteils 6 von rechts in Fig. 7. Die Flügel 23 dienen als Auflagefläche für den Nadelhalter 1, nachdem die Infusionsnadel eine gewisse Zeit in der eingeführten Stellung verbleiben muss.

Fig. 8 und 9 zeigen bei einem Aufbau nach den Fig. 5 bis 7 eine Nadelkappe 13, die mit zwei beabstandeten Haltebügeln 27 versehen ist, die über ein abgebogenes freies Ende 27' an den Flügeln 23 eingehängt werden, wie Fig. 8 zeigt. Bei dieser Ausführungsform liegt das proximale Ende der Nadelkappe 13 zweckmäßigerweise am Vorderende des Schutzelementes 3 an, wie Fig. 8 zeigt, damit das Schutzelement 3 in seiner Bereitstellung gehalten wird.

Es ist aber auch möglich, am proximalen Ende der Nadelkappe 13 einen haubenförmigen Ansatz vorzusehen, der auf der Vorderseite des haubenförmigen Abschnitts 26 anliegt.

Bei den beschriebenen Ausführungsformen ist jeweils ein Schutzelement in der Form eines Federclips mit sich kreuzenden Armen wiedergegeben. Es kann aber auch eine andere Ausführungsform eines Schutzelementes in Verbindung mit dem Griffteil 6 verwendet werden.

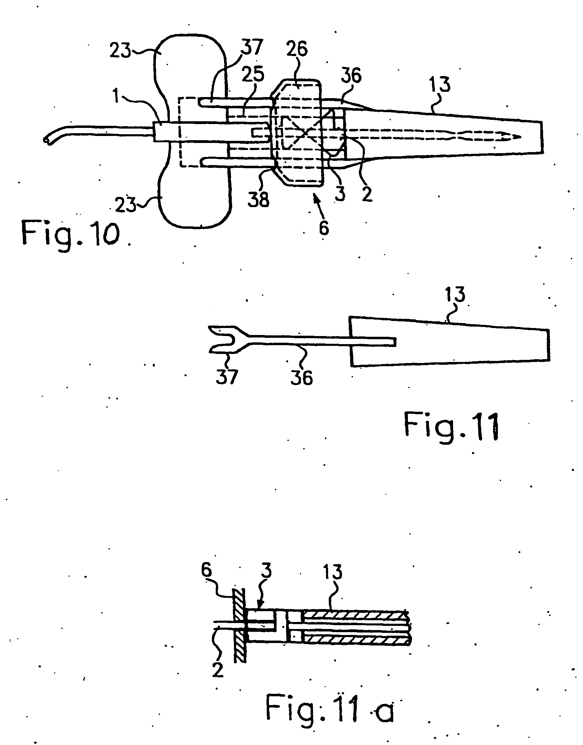

Fig. 10 zeigt eine weitere Ausführungsform der Vorrichtung nach den Fig. 5 bis 7. Die Nadelkappe 13 ist hierbei anstelle der Einhängebügel 27 auf den beiden Seiten mit jeweils einer Verlängerungsstrebe 36 versehen, die am freien Ende einen gabelförmigen Abschnitt 37 zum Einstecken an den Flügeln 23 des Nadelhalters aufweist (Fig. 11). Diese beiden beabstandeten Streben 36 erstrecken sich durch entsprechend groß ausgebildete Öffnungen 38 in dem Haubenabschnitt 26 des Griffteils 6, so dass die gabelförmigen Steckabschnitte 37 ohne weiteres durch diese Öffnungen 38 abgezogen werden können. Beim Abziehen der Nadelkappe 13 wird der Nadelhalter 1 gehalten, wobei das Griffteil 6 nicht bewegt wird.

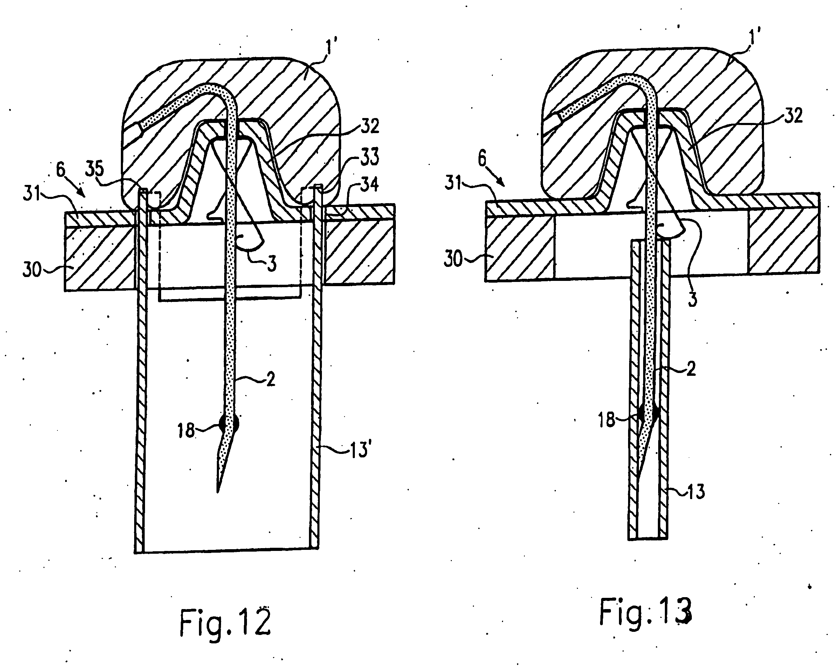

Fig. 12 zeigt eine Ausgestaltung in Verbindung mit einer Huber-Nadel 2, die mittels eines abgebogenen Abschnitts in einem Nadelhalter 1' gehalten ist und zum senkrechten Einführen bei einer Injektion vorgesehen ist. Mit 30 ist ein vorzugsweise aus Schaumstoffmaterial bestehendes Auflageteil bezeichnet, das mit einer Klebefläche zur besseren Fixierung auf der Haut des Patienten versehen ist. Zwischen dem Auflageteil 30 und dem Nadelhalter 1' ist ein schildförmiges Griffteil 6 angeordnet, das über einen flanschartigen Bereich 31 auf dem Auflageteil 30 aufliegt und sich über einen topfförmigen Mittelabschnitt 32 in eine entsprechende Vertiefung des Nadelhalters 1' erstreckt. In diesem topfförmigen Mittelteil 32 ist das Schutzelement 3 angeordnet.

Beim Herausziehen der Nadel wird das Griffteil 6 auf dem Auflageteil 30 gehalten, während der Nadelhalter 1' abgezogen wird. Hierbei wird das Schutzelement 3 zur Nadelspitze verschoben, bis es an der Nadelkröpfung 18 zum Anliegen kommt, während gleichzeitig die beiden sich kreuzenden Arme des Schutzelementes 3 die Nadelspitze übergreifen und abdecken. Hierauf kann das Griffteil 6 vom Auflageteil 30 oder zusammen mit diesem abgenommen werden. Griffteil 6 und Auflageteil 30 können über eine Klebeschicht auch miteinander verbunden sein.

Die Seitenwände des topfförmigen Mittelteils 32 sind vorzugsweise kegelförmig geneigt, so dass das Griffteil 6 selbst nicht abgezogen, sondern nur gedrückt werden kann.

Fig. 12 zeigt eine Nadelkappe 13' mit einem rohrförmigen Abschnitt, an dessen proximalem Ende diametral gegenüberliegende Wandabschnitte 33 vorstehen, die über teilkreisförmige Schlitze 34 in dem Flansch 31 des Griffteils 6 in entsprechend teilkreisförmige Nuten 35 im Nadelhalter 1' eingesteckt sind. Die gebogenen Wandabschnitte 33 sind lose durch die gebogenen Schlitze 34 im Flansch 31 des Griffteils 6 geführt und mit Preßsitz in die Nuten 35 des Nadelhalters 1' eingesteckt.

Wie bei den anderen Ausführungsformen einer Nadelkappe 13 kann auch die Nadelkappe 13' in Fig. 12 an der distalen Seite geschlossen ausgebildet sein.

Fig. 13 zeigt eine Ausgestaltung mit Huber-Nadel 2 entsprechend Fig. 12, wobei eine Nadelkappe 13 mit kleinerem Innendurchmesser auf die Nadel 2 aufgeschoben ist, wobei die Nadelkappe 13 durch Reibung an dem abgewinkelten Vorderende auf der Nadel gehalten wird. Diese Nadelkuppe 13 in Fig. 13 kann mit radial abstehenden und diametral gegenüberliegenden Flächenabschnitten 52 versehen sein, durch welche die Handhabung verbessert und die schlauchförmige Nadelkappe 13 versteift wird.

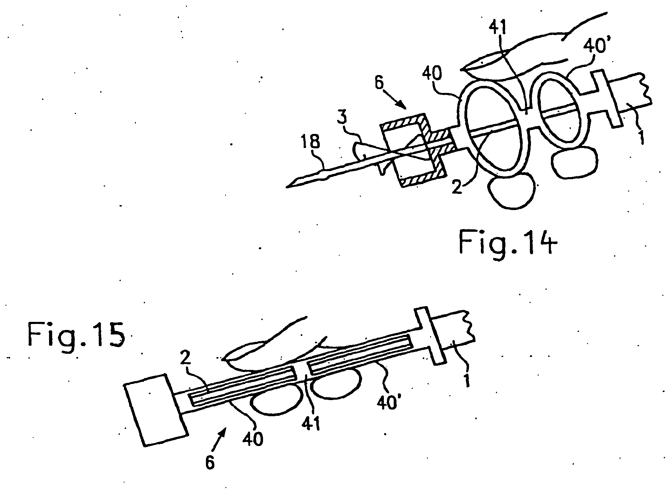

Fig. 14 und 15 zeigen eine Ausführungsform, bei der das Griffteil 6 einen verformbaren Abschnitt aufweist, mittels dem das distale Ende des Griffteils, an dem das Schutzelement 3 anliegt, in Richtung der Schutzposition an der Nadelspitze verschoben werden kann, indem der verformbare Abschnitt verformt wird. Bei dem Ausführungsbeispiel nach Fig. 14 und 15 sind zwei Paare von formbaren Bügeln 40 und 40' an dem Griffteil 6 ausgebildet, die mit den Fingern zusammengedrückt werden können, so dass sie aus der gebogenen Stellung in Fig. 14 in eine gestreckte Stellung in Fig. 15 überführt werden. Die beiden verformbaren Bügelpaare 40 und 40' sind durch einen Hülsenabschnitt 41 miteinander verbunden. Es ist auch möglich, zwischen den beiden Bügelpaaren 40 und 40' ein Element einzusetzen, das durch Druck mittels der Finger die beiden verformbaren Bügel 40 und 40' in die gestreckte Stellung nach Fig. 14 überführt.

Die Ausführungsformen nach den Fig. 1 bis 13 haben gegenüber den Ausführungsformen nach den Fig. 14 und 15 den Vorteil, dass eine größere Kanülenlänge in der Bereitstellung zur Verfügung steht, weil das Schutzelement 3 unmittelbar am Nadelhalter anliegt, während bei den Ausführungsformen nach den Fig. 14 und 15 ein aufwendigerer Aufbau des Griffteils 6 zwischen Schutzelement 3 und Nadelhalter 1 vorgesehen ist, wodurch die zur Verfügung stehende Kanülenlänge eingeschränkt wird.

Bei allen Ausführungsformen wird als Schutzelement 3 bevorzugt ein Nadelclip aus Metall verwendet, dessen sich kreuzende Arme von gegenüberliegenden Seiten eines proximalen Wandabschnitts ausgehen, der ein Loch für den Durchtritt der Nadel aufweist, wobei der Lochdurchmesser kleiner ist als die maximale Querabmessung der Nadel an der Quetschung 18, so dass der Nadelclip durch den im Durchmesser vergrößerten Abschnitt 18 in der Schutzstellung an der Nadelspitze gehalten wird. Die sich kreuzenden Arme, die beiderseits der Nadel 2 verlaufen, wie Fig. 11a zeigt, weisen am distalen Ende einen etwa auf die Breite der Rückwand verbreiterten Endabschnitt auf, der in der Ausgangsstellung auf dem Außenumfang der Nadel unter elastischer Vorspannung aufliegt und bei Erreichen der Nadelspitze durch Federwirkung in die Schutzstellung bewegt wird, in der die beiden verbreiterten Endabschnitte die Nadelspitze übergreifen. Hierzu sind die distalen Enden der Arme, wie die Seitenansichten zeigen, in Längsrichtung etwas versetzt zueinander bzw. die Arme unterschiedlich lang, so dass sichergestellt ist, dass die beiden abgewinkelten Endabschnitte der Arme die Nadelspitze übergreifen. Zumindest am längeren Arm ist der Endabschnitt am freien Rand nach innen gebogen, um die Abdeckung der Nadelspitze auch dann zu gewährleisten, wenn versucht werden sollte, den Nadelclip aus der Schutzstellung auf der Nadel zurückzuschieben, wobei sich der nach innen abgebogene Endabschnitt an der Nadelspitze verhakt. Der Nadelclip kann insgesamt sehr kompakt ausgebildet werden und nur etwa 7 mm lang sein.

## Patentansprüche

1. Schutzvorrichtung für eine Injektions- bzw. Infusionsnadel (2), umfassend
einen Nadelhalter (1) am proximalen Ende der Nadel,
ein Schutzelement (3) für die Nadelspitze in Form eines Federclips, das auf dem Schaft der Nadel verschiebbar ist, und
ein Griffteil (6) zwischen Schutzelement und Nadelhalter zum Verschieben bzw. Halten des Schutzelements (3), in dessen Hohlraum das Schutzelement aufgenommen ist,
wobei das Schutzelement einen proximalen Wandabschnitt mit einem Loch für den Durchtritt der Nadel aufweist, und
eine Eingriffsvorrichtung zwischen Nadelumfang und Schutzelement vorgesehen ist, durch die das Schutzelement daran gehindert ist, über die Nadelspritze hinaus verschoben zu werden, wobei
das Loch im proximalen Wandabschnitt des Schutzelements die Eingriffseinrichtung mit einem Wulst, Vorsprüngen, Nadelquetschung oder Nadelkröpfung (18) bildet, und
das Griffteil (6) das Schutzelement (3) am proximalen Ende derart übergreift, dass es durch das Schutzelement daran gehindert ist, über die Nadelspitze hinaus verschoben zu werden, und
**dadurch gekennzeichnet, dass**
das Schutzelement (3) Arme aufweist, die von dem proximalen Wandabschnitt in distaler Richtung ausgehen und sich kreuzen.

2. Schutzvorrichtung nach Anspruch 1, wobei am distalen Ende eines Armes des Schutzelements (3) ein etwa auf die Breite des proximalen Wandabschnitts verbreiterter Endabschnitt zur Abdeckung der Nadelspritze in einer Schutzstellung vorgesehen ist, der in einer Ausgangsstellung auf dem Nadelschaft unter elastischer Vorspannung aufliegt

3. Schutzvorrichtung nach Anspruch 1, wobei die Arme des Schutzelementes unterschiedlich lang sind.

4. Schutzvorrichtung nach einem der Ansprüche 2 und 3, wobei der Endabschnitt nach innen gebogen ist

5. Schutzvorrichtung nach einem der Ansprüche 2 bis 4, wobei der Endabschnitt am längeren Arm ausgebildet ist.

6. Schutzvorrichtung nach einem der Ansprüche 1 bis 5, wobei die Arme des Schutzelements Abschnitte aufweisen, die weniger breit sind als der proximale Wandabschnitt und der distale Endabschnitt

7. Schutzvorrichtung nach einem der Ansprüche 1 bis 6, wobei das Schutzelement als Clip aus Metall ausgebildet ist

8. Schutzvorrichtung nach einem der vorhergehenden Ansprüche, wobei das Griffteil (6) das Schutzelement seitlich vollständig umschließt

## Claims

1. A protective device for an injection or infusion needle (2), comprising
a needle hub (1) at the proximal end of the needle,
a protective element (3) for the needle tip in the form of a spring clip, the protective element being displaceable on the needle shaft, and
a grip part (6) between the protective element and the needle hub for displacing or holding the protective element (3), the protective element being housed in the hollow space thereof,
wherein the protective element has a proximal wall portion with a hole for the passage of the needle, and
an engaging means is provided between the needle circumference and the protective element, by which the protective element is prevented from being displaced beyond the needle tip, wherein
the hole in the proximal wall portion of the protective element forms the engagement means having a bead, projections, needle crimp or needle bulge (18), and
the grip part (6) overlaps the protective element (3) at the proximal end such that it is prevented by the protective element from being displaced beyond the needle tip, and
**characterized in that**
the protective element (3) has crossing arms which extend from the proximal wall portion in a distal direction.

2. The protective device according to claim 1, wherein at the distal end of one arm of the protective element (3), an end portion is provided which is broadened to approximately the width of the proximal wall portion for covering the needle tip in a protective position and which abuts on the needle shaft under elastic prestress in a ready position.

3. The protective device according to claim 1, wherein the arms of the protective element are of different lengths.

4. The protective device according to one of claims 2 and 3, wherein the end portion is bent inwards.

5. The protective device according to one of claims 2 to 4, wherein the end portion is formed at the longer arm.

6. The protective device according to one of claims 1 to 5, wherein the arms of the protective element have portions which are less broad than the proximal wall portion and the distal end portion.

7. The protective device according to one of claims 1 to 6, wherein the protective element is formed as a metal clip.

8. The protective device according to one of the preceding claims, wherein the grip part (6) completely surrounds the protective element laterally.

## Revendications

1. Dispositif de protection pour une aiguille d'injection ou de perfusion (2) comprenant
un support d'aiguille (1) sur l'extrémité proximale de l'aiguille,
un élément protecteur (3) pour la pointe d'aiguille, se présentant sous la forme d'une agrafe à ressort, qui est déplaçable sur l'axe de l'aiguille, et
une partie de préhension (6) entre l'élément protecteur et le support d'aiguille pour le déplacement ou le maintien de l'élément protecteur (3), dans l'espace creux de laquelle est logé l'élément protecteur,
dans lequel l'élément protecteur présente une section de paroi proximale avec un trou pour le passage de l'aiguille, et
il est prévu un dispositif d'engagement entre la périphérie de l'aiguille et l'élément protecteur, par lequel l'élément protecteur est empêché d'être déplacé au-delà de la pointe de l'aiguille, dans lequel
le trou dans la section de paroi proximale de l'élément protecteur forme le dispositif d'engagement avec un bourrelet, des saillies, un pincement d'aiguille ou un coude d'aiguille (18) et
la partie de préhension (6) enserre par le dessus l'élément protecteur (3) sur l'extrémité proximale, de telle sorte que l'élément de protection est empêché d'être déplacé au-delà de la pointe de l'aiguille, et
**caractérisé en ce que** l'élément protecteur (3) comporte des bras qui partent depuis la section de paroi proximale dans la direction distale et se croisent.

2. Dispositif de protection selon la revendication 1, dans lequel sur l'extrémité distale d'un bras de l'élément protecteur (3), est prévue une section d'extrémité élargie sensiblement sur la largeur de la section de paroi proximale pour recouvrir la pointe d'aiguille dans une position de protection qui, dans une position de sortie, s'appuie sur l'axe de l'aiguille en précontrainte élastique.

3. Dispositif de protection selon la revendication 1, dans lequel les bras de l'élément protecteur sont d'une longueur différente.

4. Dispositif de protection selon l'une des revendications 2 et 3, dans lequel la section d'extrémité est recourbée vers l'intérieur.

5. Dispositif de protection selon l'une des revendications 2 à 4, dans lequel la section d'extrémité est formée sur le bras le plus long.

6. Dispositif de protection selon l'une des revendications 1 à 5, dans lequel les bras de l'élément protecteur présentent des sections qui sont moins larges que la section de paroi proximale et la section d'extrémité distale.

7. Dispositif de protection selon l'une des revendications 1 à 6, dans lequel l'élément protecteur est configuré sous forme de clip en métal.

8. Dispositif de protection selon l'une des précédente revendications, dans lequel le partie de préhension (6) entoure l'élément protecteur latérale complètement.
